Europäisches Patentamt

**European Patent Office**

**Office européen des brevets**

(19)

(11) Publication number : **0 493 060 A2**

# (12) EUROPEAN PATENT APPLICATION

(21) Application number : **91311927.7**

(22) Date of filing : **23.12.91**

(51) Int. Cl.$^5$ : **C12P 13/02**, C12N 15/52,
C12N 15/70, C12N 1/20,
// (C12N1/21, C12R1:19)

(30) Priority : **25.12.90 JP 414465/90**

(43) Date of publication of application :
**01.07.92 Bulletin 92/27**

(84) Designated Contracting States :
**AT BE CH DE DK ES FR GB GR IT LI LU MC NL SE**

(71) Applicant : **TAKEDA CHEMICAL INDUSTRIES, LTD.**
**1-1, Doshomachi 4-chome**
**Chuo-ku, OSAKA (JP)**

(72) Inventor : **Miki, Hiroshi**
**E504, 14 Minatojimanakamachi 6-chome**
**Chuo-ku, Kobe Hyogo 650 (JP)**
Inventor : **Yamaguchi, Takamasa**
**10-3, Shioyakitamachi 2-chome, Tarumi-ku**
**Kobe, Hyogo 655 (JP)**
Inventor : **Hikichi, Yuichi**
**50-1, Yamadaminami**
**Suita, Osaka 565 (JP)**
Inventor : **Nogami, Ikuo**
**39-4, Uquisudai**
**Nagaokakyo, Kyoto 617 (JP)**
Inventor : **Moriya, Takeo**
**50-1, Yamadainami**
**Suita, Osaka 565 (JP)**

(74) Representative : **Laredo, Jack Joseph**
**Elkington and Fife Prospect House 8**
**Pembroke Road**
**Sevenoaks, Kent TN13 1XR (GB)**

(54) **Production method of d-pantothenic acid and plasmids and microorganisms thereof.**

(57)    A method for production of D-pantothenic acid which comprises contacting DL-pantoic acid, DL-pantolactone or a mixture thereof and β-alanine with a microorganism which is capable of producig D-pantothenic acid from DL-pantoic acid and β-alanine but substantially does not produce L-pantothenic acid.

The present invention enables stereoselective condensation reaction of inexpensive starting compounds, DL-pantoic acid (or its salt) and/or DL-pantolactone and β-alanine (or its salt), thereby to provide a method for producing D-pantothenic acid or its salts in high optical yield.

EP 0 493 060 A2

This invention relates to a new method for producing D-pantothenic acid or its salts which is important as a vitamin.

As the methods for production of D-pantothenic acid (or its salts), there have been so far known (1) a method of chemical condensation of D-pantolactone, obtained by optical resolution of DL-pantolactone, with β-alanine (or its salts) in methanol; (2) a method of producing D-pantothenic acid by hydrolysis of D-pantothenic acid ester with a microorganism or an enzyme, or a method for producing D-pantothenic acid by selective hydrolysis of the D-form compound alone from DL-pantothenic acid ester (respectively described in Japanese Patent Application Laid-open (Kokai) Nos. 228487/1989 and 228488/1989); and (3) a method for the preparation of pantothenic acid by bringing potassium pantoate, β-alanine and ATP in contact with resting cells or their enzyme in Tris buffer [Journal of Biological Chemistry, 198, p23 (1952) ; Abstracts of Papers, 176th American Chemical Society National Meeting, Division of microbial and biochemical technology, 48/1978; etc.]

In industrially manufacturing D-pantothenic acid (hereinafter referred to as D-pantothenic acid, including its salts), the method (1) mentioned above has the defect that it requires chemically complicated steps for optical resolution of DL-pantolactone, and the method (2) has the defect that it requires steps for producing D-pantothenic acid ester or DL-pantothenic acid ester from DL-pantolactone. The method (3) is disadvantageous in using expensive ATP or Tris buffer and besides the method is not practical in that the amount of the produced pantothenic acid is so small, and D-pantoic acid (or its salts) to be used as the starting compound is expensive.

Thus, there has been a great demand for the development of methods for producing D-pantothenic acid or its salts which are more practical and industrially applicable.

For the purpose of attaining production of D-pantothenic acid and its salts in an industrially advantageous manner, the present inventors had made extensive studies of the methods in which using inexpensive DL-pantoic acid or a salt thereof and/or DL-pantolactone as a starting compound, D-pantothenic acid or a salt thereof can be obtained by subjecting D-pantoic acid or a salt thereof and/or D-pantolactone alone to stereoselective condensation reaction with β-alanine. As a result, they found that unexpectedly, the above-mentioned stereoselective condensation reaction could be effected by using microorganisms, followed by further studies based on the findings, which led them to accomplishment of this invention.

The present inventors made further studies of application of gene recombinant technology to production of D-pantothenic acid and breeding of bacterial strains. As a result, they found that some bacterial strains which were transformed with plasmid harbouring genes for enzyme for synthesis of pantothenic acid (hereinafter referred to as pantothenic acid-synthesizing enzyme-coding gene) accumulated D-pantothenic acid at higher concentrations in the culture media, which culminated in completion of this invention. That is, this invention relates to a method for producing D-pantothenic acid characterized by bringing DL-pantoic acid, DL-pantolactone or a mixture thereof and β-alanine into contact with a microorganism which is capable of producing D-pantothenic acid from DL-pantoic acid and β-alanine but substantially does not produce L-pantothenic acid.

Also, this invention provides new plasmids harbouring a pantothenic acid-synthesizing enzyme-coding gene DNA and new microorganisms which are transformants of said plasmids and thus useful for producing D-pantothenic acid.

The present invention is described in detail below.

The objective D-pantothenic acid and the DL-pantoic acid and β-alanine to be used as the starting compounds may be used in their salt forms. Examples of the salts include the salts of alkali metals and alkaline earth metals. Among others, particularly the respective calcium salt, sodium salt and potassium salt are preferred. Hereinafter, when reference is made to D-pantothenic acid, DL-pantoic acid and β-alanine, this includes such salts thereof as mentioned above.

As the microorganisms to be used for the stereoselective condensation reaction of the present invention, any microorganisms can be used as long as they are capable of producing D-pantothenic acid from DL-pantoic acid and β-alanine but substantially do not produce L-pantothenic acid. In this invention, preferable are such microorganisms are substantially fail to act on L-pantoic acid but can produce D-pantothenic acid from D-pantoic acid and β-alanine. Specifically, examples of such microorganisms include microorganisms belonging to the following genera: Aquaspirillum, Arthrobacter, Aureobacterium, Bacillus, Brevibacterium, Brochothrix, Chromobacterium, Citrobacter, Corynebacterium, Curtobacterium, Enterobacter, Erwinia, Escherichia, Flavobacterium, Gluconobacter, Hafnia, Hyphomicrobium, Klebsiella, Micrococcus, Oceanospirillum, Photobacterium, Pimelobacter, Protaminobacter, Providencia, Pseudomonas, Rhodococcus, Salmonella, Serratia, Xanthomonas, Debaryomyces, Filobasidium, Kluyveromyces, and Zygosaccharomyces. As representative examples of the microorganisms which can be used most efficiently, mention can be made of Aquaspirillum metamorphum IFO 13960, Arthrobacter ureafaciens IFO 12140, Aureobacterium terregens IFO 12961, Bacillus cereus subsp. mycoides IFO 3836, Brevibacterium ammoniagenes IFO 12072, Brochothrix thermosphacta IFO 12167, Chromobacterium chocolatum IFO 3758, Citrobacter freundii IFO 13545, Corynebacterium erythrogenes IFO 12974, Curtobacterium luteum IFO 12676, Enterobacter cloacae IFO 13536, Erwinia carotovora subsp. carotovora IFO 3380, Escherichia coli IFO 14237, Flavobacterium lutescens IFO 3084, Gluconobacter suboxydans IFO 3130, Hafnia alvei IFO 3731, Hyphomicrobium indicium IFO 14233, Klebsiella pneumoniae IFO 13703, Micrococcus varians IFO 3765, Oceanospirillum multiglobuliferum IFO 13614, Photobacterium mandapamensis IFO 14169, Pimelobacter simplex IFO 12069, Protaminobacter ruber IFO 3708, Providencia rettgeri IFO 13501, Pseudomonas aureofaciens IFO 3521, Rhodococcus fascians IFO 12077, Salmonella typhimurium IFO 14193, Serratia rubidaea IFO 12973, Xanthomonas phaseoli IFO 13553, Debaryomyces castellii IFO 1359, Filobasidium floriforme IFO 1603, Kluyveromyces phaffii IFO 1672, Zygosaccharomyces bisporus IFO 1131, etc. Particularly, among others, preferred are microorganisms of Escherichia coli such as a Escherichia coli IFO14237. These are the known strains which are listed in "List of Cultures, The 8th Edition, 1988" published by the Institute for Fermentation Osaka, Japan, and are available from the Institute of Fermentation Osaka.

Also, usable are the artificiallly produced mutants from the above strains by ultraviolet ray-radiation or treatment with a chemical mutagen and the other microorganisms which are recombinated with the gene fragment essential for expression of the condensation activity or the fragment of the pantothenic acid-synthesizing enzyme-coding gene taken out from the above strains.

The pantothenic acid-synthesizing enzyme-coding genes represent **pan C** genes and are genes coding Pantothenate Synthase. Examples of the donor microorganisms of the aforesaid DNA include microorganisms belonging to the species Escherichia coli. More specific and preferred examples thereof include known such as Escherichia coli K-12 (IFO 3301), Escherichia coli (IFO 3547) and the like, which are listed on "List of Cultures The 8th Edition (1988)" published by the Institute of Fermentation Osaka, Japan, The 8th Edition (1988).

As methods for preparing the DNA fragment containing a gene coding pantothenic acid-synthesizing enzyme, mention is made of, for example, a method in which a chromosomal DNA is extracted from the donor microorganisms in accordance with a known method such as the method described in Biochim, Biophys. Acta 72 619 (1963) [H. Saito and K. Miura] or a method analogous thereto, followed by digestion with a restriction endonuclease such as EcoRI.

The thus-obtained chromosomal DNA fragment containing a pantothenic acid-synthesizing enzyme-coding gene is inserted into a vector DNA. As the vector DNA to be used in the present invention, employable are any vectors that can replicate in microorganisms belonging to the species Escherichia coli. Examples of vectors which can replicate in microorganisms belonging to Escherichia coli include plasmids such as pSC101 [Proc. Natl. Acad. Sci., U.S.A., 20, 3240 (1973)] and pBR322 [Gene, 4, 121 (1978)].

The above-mentioned ones are not limitative, and any vector DNA's, including vectors that are newly isolated or synthesized, can be used as long as they can accomplish the object of the present invention.

The above-mentioned DNA fragment containing a pantothenic acid-synthesizing enzyme-coding gene can be inserted into a plasmid vector by a known method, for example, a method as described in T. Maniatis et al., Molecular Cloning, A Laboratory Manual, Cold Spring Harbor Laboratory, Tokyo University Publishing Association (1982).

The resulting recombinant plasmid vector containing a pantothenic acid-synthesizing enzyme-coding gene can be introduced into a recipient microorganism in accordance with a known transformation method, for example, the method described in J. Mol. Biol. 53 159 (1979). Examples of said recipient microorganism include known strains such as Escherichia coli C600 Strain (IFO 14410) [Bacteriol., Rev. 36, 525 (1972)].

Among the resulting transformants, selection of the objective transformants in which the plasmid harbouring a pantothenic acid-synthesizing enzyme coding gene is introduced can be effected, for example, by selecting the strains which get to grow in culture media free from pantothenic acid, as a consequence of transformation, using pantothenic acid-requiring strains as a DNA recipient.

Selection of the objective transformants can be facilitated by using a culture medium in which strains characteristic of a marker of plasmid DNA can be selected. The thus-obtained recombinant vector DNA's containing a pantothenic acid-synthesizing enzyme-coding gene can be extracted from the transformants and introduced into other recipients. The extraction can be conducted by a known method, for example, the method described in "Molecular Cloning" (published by Tokyo University, in 1982, pp. 90-91) or a method analogous thereto. Also, after a DNA fragment containing a pantothenic acid-synthesizing enzyme-coding gene is prepared from the extracted recombinant vector DNA, the fragment can be ligated with another vector plasmid. As mentioned above, the objective transformants can be obtained by per-se known means and techniques or their analogous methods in combination. Specific examples of the transformants obtainable in the foregoing manner include Escherichia coli KE3 (IFO 15215, FERM 3563), Escherichia coli 3E5 (IFO 15216, FERM 3564), etc.

Said Escherichia coli KE3 is a strain transformed of a plasmid harbouring a pantothenic acid-synthesizing enzyme-coding gene obtained from Escherichia coli K-12 (IFO 3301), and Escherichia coli 3E5 strain is a strain transformed of a plasmid harbouring a pantothenic acid-synthesizing enzyme-coding gene obtained from Escherichia coli IFO 3547.

3

Herein, the IFO numbers represent the accession numbers of deposit at the Institute of Fermentation Osaka (17-85, 2-chome, Juso-honmachi, Yodogawa-ku, Osaka-City Oska, Japan) and the FERM BP-numbers represent the accession numbers of deposit at the Fermentation Research Institute, Agency of Industrial Science and Technology, Japan (FRI) (1-3, 1-chome, Tsukuba-City, Ibaragi, Japan).

As the culturing methods of the aforementioned strains, usable are standing culture, shaking culture (e.g. rotary shaking culture) and aeration stirring culture. As culture media to be used, employable are any culture media having an ordinary composition as long as the microorganism used can grow in it. As carbon sources, there can be used one or a combination of carbohydrates, oils and fats, fatty acids, organic acids and alcohols which the microorganism used can grow on. Usually, in this invention, preferably used are culture media containing carbohydrates such as glucose, sucrose, etc. Examples of the nitrogen sources include organic nitrogen sources such as peptone, soybean meal, cotton seed flour, corn steep liquor, yeast extract, meat extract, malt extract and urea and inorganic nitrogen sources such as ammonium sulfate, ammonium chloride, ammonium nitrate and ammonium phosphate, and they are used singly or in a mixture. As phosphorus sources, monobasic potassium phosphate, dibasic potassium phosphate, etc. are preferably used. Additives other than carbon sources, nitrogen sources and phosphorus sources which can be used for said media include substances essential for growth or growth-promoting substances such as metal salts (e.g. magnesium sulfate), amino acids (e.g. aspartic acid, valine, alanine, leucine, tyrosine, phenylalanine, tryptophan), vitamins (e.g. vitamin $B_1$, biotin, pyridoxin, nicotinic acid). If desired, for the purpose of controlling pH in the course of culture, there can be added basic substances such as sodium hydroxide, potassium hydroxide, ammonia and calcium carbonate. Also, the addition of antifoaming agents to control foam may be desirable in some cases. The above additives may be added, if desired, in the midst of culture. Aeration or oxygenation may also be desirable to improve culturing. The culture temperature may be selected depending on the temperature suitable for growth of the microorganism used and is, in general, advantageously in the range from 15°C to 65°C, preferably from 25°C to 40°C. The culture time is extended to a time sufficient for the growth of the microorganism used and usually ranges from 5 to 240 hours, preferably from 10 to 170 hours.

DL-pantoic acid as a starting compound can be easily prepared by opening the lactone ring of DL-pantolactone by adding thereto a basic substance such as sodium hydroxide, potassium hydroxide and ammonia. DL-pantolactone may be added directly to the culture liquid or reaction mixture, whereby the lactone ring is opened to yield DL-pantoic acid in the culture broth or reaction mixture. As the method for bringing DL-pantoic acid and/or DL-pantolactone and β-alanine as starting compounds into contact with the aforesaid microorganisms, employable are the method in which the starting compounds are added at a suitable time before the initiation of culture of the strains or in the midst of culturing, and the method in which the starting compounds are added to the processed bacterial cells at a suitable time. "The processed bacterial cells" include washed bacterial cells of the culture product obtainable by culture of the bacteria, acetone powders, immobilized bacterial cells of the culture product in polyacrylamide gel or K-carrageenan and the like. The starting compounds may be added in a solution or a suspension in an appropriate solvent such as water, or in powdery forms at one time or continuously or intermittently over a certain period. In the reaction in which bacterial cells are contacted with the starting compounds, the concentrations of DL-pantoic acid or DL-pantolactone and β-alanine in the culture broth or reaction mixture are preferably 0.1-40% (w/v) and 0.03-20% (w/v), respectively, more preferably 0.5-30% (w/v) and 0.15-9% (w/v) respectively. Usually, DL-pantoic acid or DL-pantolactone and β-alanine are reacted preferably in a molar ratio of about 2:1 in accordance with the stoichiometrical ratio. Preferably, carbon sources (e.g. carbohydrate, oils and fats, fatty acids, organic acids, alcohols) on which the microorganisms grow are added together with the starting compounds to raise the reaction activity in the bacterial cells, and minerals, vitamins and naturally occurring substances such as peptone, corp steep liquor and yeast extract are added to promote the reaction. Particularly, the addition or carbohydrates such as glucose and sucrose is preferred.

When D-pantothenic acid in the free form is obtained in accordance with the foregoing reaction, it can be converted into its salts by a conventional method, and, conversely, when a salt of D-pantothenic acid is obtained by the above reaction, it can be converted into its free form also by a conventional method.

The D-pantothenic acid or its salts resulting from the above-mentioned reaction can be isolated and collected in a conventional manner. For example, after the removal of the bacterial cells from the reaction product, the remaining reaction product is subjected to a known procedure or to a combination of two or more known procedures such as treatment with ion-exchange resin, adsorption treatment with activated charcoal, etc., fractional crystallization or distillation, sublimation. The pantoic acid remaining in the reaction mixture is also subjected to one or more treatments such as that with an ion-exchange resin, that with activated charcoal, that with an acid, solvent extraction, and racemization treatment, whereby racemic D-pantolactone can be recovered and used again as a starting compound.

As one of the specific examples of the methods for isolation and collection of calcium D-pantothenate, there may be mentioned the following method. After the bacterial cells are removed from the reaction broth by fil-

4

tration, the filtrate is subjected to a cation-exchange resin [e.g. Diaion® PK-216 (H type) (manufactured by Mitsubishi Chemical Industries Ltd.)], whereby removal of cations in the reaction broth can be effected and also the cyclization reaction of pantoic acid into pantolactone can be carried out. The thus-obtained eluate is subjected to an anion-exchange resin [e.g. Diaion® WA-30 (OH type), Diaion® WA-21 (OH type), Diaion® PA-412 (OH type) (manufactured by Mitsubishi Chemical Industries Ltd.)], thereby to remove acidic impurities which are of acidity higher than D-pantothenic acid.

After a calcium compound such as calcium hydroxide, calcium oxide or calcium carbonate is added to the obtained eluate, the mixture is concentrated under reduced pressure, to reduce water content to a suitable extent. Removal of water under reduced pressure can be effected by means of azeotropy, adding a suitable solvent which allows azeotropy with water (e.g. isopropanol, ethanol). Thereafter, a suitable solvent such as a lower alcohol (e.g. isopropanol, methanol) is added, followed by crystallization and filtration to obtain crystals of calcium DL-pantothenate. In the course of the above-mentioned process, the pantoic acid in the culture broth is converted into pantolactone. The pantolactone in the mother liquor of filtration can be recovered and subjected to treatment for racemization to DL-pantolactone by a conventional method.

The present invention enables the stereoselective condensation reaction of inexpensive DL-pantoic acid and/or DL-pantolactone with β-alanine, whereby D-pantothenic acid or its salts can be produced in high optical yield. The present invention is hereinafter described more specifically by illustrating working examples, which are merely exemplary but not limitative on the scope of the present invention.

The DL-pantoic acid used in the following examples was prepared by dissolving DL-pantolactone and sodium hydroxide in equimolar amounts in ion-exchanged water, followed by three hours' stirring at room temperature.

The quantitative determination of D-pantothenic acid was effected by high performance liquid chromatography [column: Shimadzu SCR101H (7.9 mm ∅ x 30 cm); mobile phase: 0.008N sulfuric acid; flow rate 0.8 ml/min.; detection: differential refractometer] and a microbiological quantitative method (Bioassay) [test bacterium: Lactbacillus plantarum IFO 3070; culture medium: marketed pantothenic acid-quantification medium (manufactured by DIFCO)].

The optical purities of D-pantothenic acid in the culture broth were measured in the following manner. After completion of culture, the culture broth was centrifuged and the obtained supernatant was subjected to the column of a cation-exchange resin, Diaion PK-216 (H type) (manufactured by Mitsubishi Chemical Industries Ltd.), and the obtained eluate was subjected to the column of an anion-exchange resin, Amberlite IRA-68 ($CH_3COO$ type) (manufactured by Japan Organo Co. Ltd.) for adsorption, followed by elution with 0.2N acetic acid, whereby pantoic acid and pantothenic acid are completely separately eluted from the column to give an aqueous solution of pantolactone and an aqueous solution of pantothenic acid, respectively. In accordance with the method described in Journal of Chromatography, 474, 405 (1989), the aqueous solution of pantothenic acid was treated in 2 ml of 0.5N NaOH under heating at 70°C for 30 min. and thereby decomposed into pantoic acid and β-alanine, whereafter the ration of D-pantoic acid and L-pantoic acid was measured by high performance liquid chromatography (column: Mitsubishi Chemical MCI GEL CRS 10W) (4.6 mm ∅ x 50 mm); mobile phase: 2 mM $CuSO_4$/acetonitrile (9/1); flow rate: 0.5 ml/min.; detection: UV254 nm). Thus, the optical purity of D-pantothenic acid in the culture broth was calculated. The culture purity was represented as enantiomer excess (e.e.) indicating the percentage of the excess portion of the D-form. The conversion yield was shown by the molar ratio of the accumulated D-pantothenic acid to the D-pantoic acid in the added DL-pantoic acid.

Example 1

To a 200 ml-ribbed Erlenmeyer flask containing 20 ml of a sterilized seed culture medium composed of 5% glucose, 3% corn steep liquor, 0.2% ammonium sulfate, 0.01% monobasic potassium phosphate, 0.03% dibasic potassium phosphate, 0.01% magnesium sulfate and 1% calcium carbonate (pH7.0) was inoculated one platinum loopful of the strains as shown in Tables 1 and 2 grown on a slant medium. Rotary shaking culture was conducted at 30°C for 24 hours at 220 rpm. The seed culture broth (1 ml) was transplanted to a 200 ml-ribbed Erlenmeyer flask containing 20 ml of a sterilized culture medium composed of 9% glucose, 3% corn steep liquor, 0.5% polypeptone, 0.5% yeast extract, 0.5% ammonium sulfate, 0.01% monobasic potassium phosphate, 0.03% dibasic potassium phosphate, 0.01% magnesium sulfate and 1% calcium carbonate (pH7.0), which was subjected to rotary shaking culture at 30°C at 220 rpm. Eight hours after initiation of culture, DL-pantoic acid and β-alanine were added at concentrations of 2% (w/v) and 0.601% (w/v) respectively to the flask, followed by a further 64 hours' culture. The amounts of the accumulated D-pantothenic acid in the culture broth after termination of culture were measured by the high performance liquid chromatography method (HPLC) and the microbiological quantification method (Bioassay) and the results are shown together with the conversion yields in Table 1.

Table 1

| Strain | IFO | Amount of accumulated D-pantothenic acid (%W/V) | | Conver- sion Yield (%) |
|---|---|---|---|---|
| | | HPLC | Bio-assay | |
| Aquaspirillum metamorphum | 13960 | 0.58 | 0.59 | 39.2 |
| Arthrobacter ureafaciens | 12140 | 0.95 | 0.98 | 64.2 |
| Aureobacterium terregens | 12961 | 0.12 | 0.23 | 8.1 |
| Bacillus cereus subsp. mycoides | 3836 | 0.76 | 0.80 | 51.4 |
| Brevibacterium ammoniagenes | 12072 | 1.09 | 1.12 | 73.7 |
| Brochothrix thermosphacta | 12167 | 0.34 | 0.36 | 23.0 |
| Chromobacterium chocolatum | 3758 | 0.54 | 0.55 | 36.5 |

| | | | | |
|---|---|---|---|---|
| Citrobacter freundii | 13545 | 0.60 | 0.66 | 40.5 |
| Corynebacterium erythrogenes | 12974 | 1.25 | 1.27 | 84.5 |
| Curtobacterium luteum | 12676 | 1.09 | 1.10 | 73.7 |
| Enterobacter cloacae | 13536 | 1.20 | 1.21 | 81.1 |
| Erwinia carotovora subsp. carotovora | 3380 | 1.06 | 1.06 | 71.6 |
| Escherichia coli | 14237 | 1.46 | 1.46 | 98.7 |
| Flavobacterium lutescens | 3084 | 0.63 | 0.65 | 42.6 |
| Gluconobacter suboxydans | 3130 | 0.25 | 0.27 | 16.9 |
| Hafnia alvei | 3731 | 0.96 | 0.99 | 64.9 |
| Hyphomicrobium indicium | 14233 | 0.22 | 0.34 | 14.9 |
| Klebsiella pneumoniae | 13703 | 0.18 | 0.25 | 12.2 |
| Micrococcus varians | 3765 | 1.04 | 1.07 | 70.3 |
| Oceanospirillum multiglobuliferum | 13614 | 0.28 | 0.29 | 18.9 |
| Photobacterium mandapamensis | 14169 | 0.26 | 0.32 | 17.6 |
| Pimelobacter simplex | 12069 | 0.49 | 0.49 | 33.1 |
| Protaminobacter ruber | 3708 | 0.17 | 0.24 | 11.5 |
| Providencia rettgeri | 13501 | 1.03 | 1.07 | 69.6 |

| | | | | |
|---|---|---|---|---|
| Pseudomonas aureofaciens | 3521 | 1.01 | 1.11 | 68.3 |
| Rhodococcus fascians | 12077 | 1.28 | 1.30 | 86.5 |
| Salmonella typhimurium | 14193 | 1.00 | 1.04 | 67.6 |
| Serratia rubidaea | 12973 | 1.15 | 1.18 | 77.7 |
| Xanthomonas phaseoli | 13553 | 0.53 | 0.58 | 35.8 |

Example 2

In a 200 ml-Erlenmeyer flask containing 20 ml of a sterilized seed culture medium composed of 5% glucose, 2% corn steep liquor; 1% malt extract, 0.5% ammonium sulfate, 0.02% magnesium sulfate and 1% calcium carbonate (pH 6.0) was inoculated one platinum loopful of the strains as shown in Table 2 grown on a slant medium, which was subjected to rotary shaking culture at 28°C for 24 hours at 220 rpm. The seed culture broth (1 ml) was transplanted to a 200 ml-Erlenmeyer flask containing 20 ml of a sterilized culture medium composed of 9% glucose, 2% corn steep liquor, 1% yeast extract, 0.5% ammonium sulfate, 0.02% magnesium sulfate and 1% calcium carbonate (pH 6.0), followed by rotary shaking culture at 30°C at 220 rpm. At the time of 24 hours' lapse after initiation of culture, DL-pantoic acid 3% (w/v) and β-alanine 0.902% (w/v) were added, and culture was further continued until 72 hours' lapse. In Table 2 are shown the results of the amounts of the accumulated D-pantothenic acid in the culture broth after termination of culture as obtained by measurement in the high performance liquid chromatography method (HPLC) and in the microbiological quantification method (Bioassay), and the conversion yield.

## Table 2

| Strain | IFO | Amount of accumulated D-pantothenic acid (%W/V) | | Conversion Yield (%) |
|---|---|---|---|---|
| | | HPLC | Bio-assay | |
| Debaryomyces castellii | 1359 | 0.42 | 0.46 | 18.9 |
| Filobasidium floriforme | 1603 | 0.58 | 0.59 | 26.1 |
| Kluyveromyces phaffii | 1672 | 0.76 | 0.77 | 34.2 |
| Zygosaccharomyces bisporus | 1131 | 0.71 | 0.78 | 32.0 |

Example 3

In a 200 ml-ribbed Erlenmeyer flask containing 20 ml of a sterilized first seed culture medium composed of 5% glucose, 5% corn steep liquor, 0.5% ammonium sulfate, 0.01% monobasic potassium phosphate, 0.03% dibasic potassium phosphate, 0.01% magnesium sulfate and 1% calcium carbonate (pH 7.0) was inoculated one platinum loopful of Escherichia coli IFO 14237 grown on a slant culture medium, which was subjected to rotary shaking culture at 30°C at 220 rpm for 24 hours. The thus-obtained first seed culture broth (20 ml) was transplanted to a 1000 ml-Erlenmeyer flask containing 200 ml of a sterilized second seed culture medium having the same composition, which was subjected to rotary shaking culture at 30°C at 220 rpm for 24 hours. The resulting second seed culture broth (125 ml) was transplanted to a 5 $\ell$-jar fermentator containing 2.5 $\ell$ of a sterilized culture medium composed of 7% glucose, 5% corn steep liquor, 1% ammonium sulfate, 0.02% monobasic potassium phosphate, 0.05% dibasic potassium phosphate, 0.01% magnesium sulfate, and 1.5% calcium carbonate (pH 7.0), followed by 24 hours' aeration (0.8 vol/vol/min.) stirring (1000 rpm) culture at 30°C. A mixture (1.8 $\ell$) of the bacterial cells collected from the resulting culture broth, 100 g of glucose, 112.5 g of DL-pantoic acid and 33.83 g of β-alanine was reacted in a 5 $\ell$-jar fermentator under aeration (0.8 vol/vol/min) and stirring (1000 rpm). Over an 18-hour period starting at initiation of reaction, an aqueous solution containing 125 g of glucose, 112.5 g of DL-pantoic acid and 33.75 g of β-alanine was continuously added. Over a 24-hour period starting 18 hours after initiation of reaction, an aqueous solution of 50 g of glucose was continuously added. After 68 hours' aeration and stirring, the final amount of the resulting broth came to 2.50 $\ell$, the concentration of D-pantothenic acid in the reaction broth was 65.6 mg/ml and the optical purity was 100% (e.e.) and the conversion yield was 98.5%.

Example 4

A platinum loopful of the strains of Escherichia coli IFO 14237 grown on a slant culture medium was inoculated in a 200 ml-ribbed Erlenmeyer flask containing 20 ml of a sterilized first seed culture medium composed of 5% glucose, 5% corn steep liquor, 0.5% ammonium sulfate, 0.01% monobasic potassium phosphate, 0.03% dibasic potassium phosphate, 0.01% magnesium sulfate and 1% calcium carbonate (pH 7.0), followed by rotary shaking culture at 30°C for 24 hours at 220 rpm. To a 1000 ml-Erlenmeyer flask containing 200 ml of a sterilized second seed culture medium of the same composition was transplanted 20 ml of the first seed culture broth, and rotary shaking culture was conducted at 30°C for 24 hours at 220 rpm. The second seed culture product (125 ml) was transplanted to a 5 $\ell$-jar fermentator containing 1.8 $\ell$ of a sterilized culture medium composed of 7% glucose, 5% corn steep liquor, 1% ammonium sulfate, 0.02% monobasic potassium phosphate, 0.05% dibasic potassium phosphate, 0.01% magnesium sulfate and 1.5% calcium carbonate (pH 7.0), followed by culture under aeration (0.8 vol/vol/min) and stirring (1000 rpm) at 30°C. Over a 43-hour period starting 5 hours after initiation of culture, a sterilized aqueous solution containing 125 g of glucose, 300 g of DL-pantoic acid, and 90.21 g of β-alanine was continuously added. Over a 24-hour period starting 48 hours after initiation of culture, a sterilized aqueous solution containing 100 g of glucose was continuously added. Ninety-six hours' culture resulted in giving 2.50 $\ell$ of the culture broth, in which the concentration of D-pantothenic acid was 87.91 mg/ml, optical purity was 100% e.e. and the conversion yield was 99.0%.

Example 5

i) Preparation of chromosomal DNA

Escherichia coli K-12 (IFO 3301) or Escherichia coli IFO 3547 strain which is capable of producing D-pantothenic acid was inoculated in 1 $\ell$ of L broth (composed of 1.0% bactotryptone, 0.5% yeast extract and 0.5% sodium chloride), followed by one night culture at 37°C. From the resulting bacterial cells, 3 mg (in the final amount) of the chromosomal DNA was obtained in accordance with the method by Saito [Biochim. Biophys. Acta, 72, 619 (1963)].

ii) Insertion of chromosomal DNA into vector plasmid pBR322

The following experiments were carried out in accordance with the experimental procedures described in "Molecular Cloning" (published by Tokyo University Publishing Association) by Maniatis et al.

After 10 μg of the chromosomal DNA as obtained in i) mentioned above and pBR322 (manufactured by Nippon Gene) were cleaved respectively with a restriction endonuclease EcoRI (manufactured by Nippon Gene), they were mixed and ligated with DNA ligase (manufactured by Nippon Gene) derived from T4 phage.

iii) Cloning of a pantothenic acid-synthetic enzyme-coding gene

By the competent cell method, transformation was carried out. That is, competent cells were prepared, using a D-pantothenic acid-requiring strain A4C strain (IFO 15251, FERM BP3677) derived from Escherichia coli C600 (IFO 14410) by treatment with N-methyl-N′nitro-N-nitrosoguanidine for mutation, and the plasmid DNA's prepared in ii) mentioned above were added to a suspension of the competent cells so that they may be incorporated therein for transformation. A suspension of the transformants was spread onto a plate M-9 agar culture medium (composed of 0.6% diabasic sodium phosphate, 0.3% monobasic sodium phosphate, 0.1% ammonium chloride, 0.05% sodium chloride, 1 mM magnesium sulfate, 1 mM calcium chloride, 0.5% glucose, 10 μg/ml of vitamin $B_1$, 50 μg/ml of threonine, 50 μg/ml of leucine and 1.5% agar, pH 7.2) containing 50 μg/ml of ampicillin sodium, followed by 2 day's culture at 37°C. From the resulting colony on the plate culture medium, a transformant having ampicillin resistance and ability to produce D-pantothenic acid was obtained. The A4C strain transformed by the plasmid harbouring an inserted DNA fragment derived from the chromosomal DNA of the Escherichia coli K-12 strain (IFO 3301) was designated as the KE2 strain (IFO 1525, FERM BP3679), and the A4C strain transformed by the plasmid harbouring an inserted DNA fragment obtained from the chromosomal DNA of Escherichia coli IFO 3547 strain was designated as KE5 strain (IFO 15253, FERM BP3678).

iv) Extraction of the plasmid from the transformant

In accordance with the method described in "Molecular Cloning" (published by Tokyo University, pp 90-91), from 1 ℓ each of the culture broths of the transformants, Escherichia coli KE2 strains and KE5 strains, 500 μg and 600 μg respectively of the plasmids were finally obtained, and the plasmids were named pFV3 and pFV5, respectively.

v) Analysis of the plasmids, pFV3 and pFV5

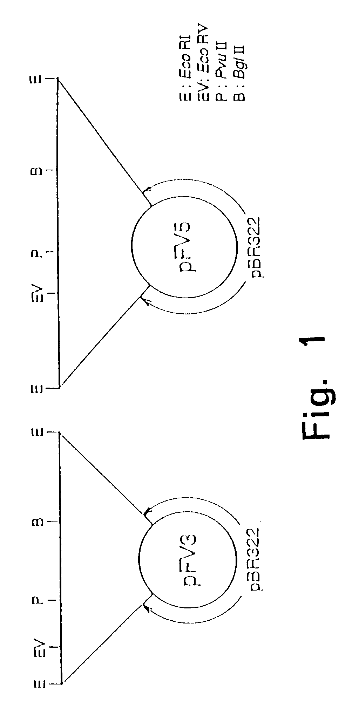

From the patterns of agarose gel electrophoresis of the fragments obtained by cleaving pFV3 and pFV5 with various restriction endonucleases based on the molecular weights of Hind III digested fragments of λ phage DNA (manufactured by Nippon Gene), the restriction maps as shown in Figure 1 were obtained. It was found that pFV3 was a recombinant plasmid with a DNA fragment of about 2.0kb inserted at the EcoRI site therein and pFV5 was a recombinant plasmid with a DNA fragment of about 2.5Kb inserted at the EcoRI site therein.

vi) Re-transformation

Using the aforementioned plasmid DNA, the pantothenic acid-requiring strain, A4C, induced from an Escherichia coli C600 strain by treatment with N-methyl-N′-nitro-N-nitrosoguanidine for mutation was subjected to re-transformation. The resulting transformants got to be able to grow on the above-mentioned M-9 agar culture medium, and therefrom it was confirmed that both pFV3 and pFV5 possessed a Pantothenate Synthase gene.

Furthermore, for the purpose of confirming the influence of the transformants on pantothenic acid-producing capability, Escherichia coli K-12 was transformed using pFV3 and Escherichia coli IFO 3547 was transformed using pFV5.

The obtained transformants were named respectively Escherichia coli KE3 (IFO 15215, FERM BP3563) and Escherichia coli 3E5 (IFO 15216, FERM BP3564).

Example 6

In 20 ml-Erlenmeyer flasks containing 20 ml respectively of a sterilized first seed culture medium having the composition as shown in Table 3 were inoculated a platinum loopful of each of Escherichia coli K-12 (IFO 3301) and KE3 (IFO 15215, FERM 3563) grown on a slant culture medium, followed by 24 hours' rotary shaking culture at 30°C at 220 rpm. To 1000 ml Erlenmeyer flasks containing 200 ml respectively of a sterilized second seed culture medium having the same composition were transplanted 200 ml of each of the first seed culture broths, followed by 24 hours' rotary shaking culture at 30°C at 220 rpm. The respective second seed culture broth (125 ml) was transplanted to a 5ℓ–jar fermentor containing 2.0 l of a sterilized culture medium comprising 125 g of glucose, 5 g corn steep liquor, 30 g of ammonium sulfate, 1.25 g monobasic potassium phosphate, 2.5 g of dibasic potassium phosphate, 0.5 g of magnesium sulfate and 37.5 g of calcium carbonate (pH 7.0), followed by culture at 37°C with aeration (0.8 Vol/Vol/Min) while stirring (900 rpm).

Reduction in concentration of dissolved oxygen with growth of the microorganisms was compensated by stirring and aeration. Over a 20-hour period starting 15 hours after culture initiation, a sterilized aqueous solution containing 250 g of glucose, 250 g of DL-pantoic acid and 75 g of β-alanine was continuously added.

After 48 hours' culture, the final volume of each of the culture broths was 2.5 $\ell$. The amount of the accumulated D-pantothenic acid was 59.2g/l in the culture broth of Escherichia coli K-12 (IFO 3301), whereas the amount of the accumulated D-pantothenic acid was 74.5 g/$\ell$ in the culture broth of Escherichia coli KE3.

The molar yield based on DL-pantoic acid in the former was 79%, whereas that in the latter was 99.3%.

## Table 3

### Composition of Liquid Culture Medium (pH 7.0)

| Composite | Concentration (%) |
| --- | --- |
| Glucose | 5.0 |
| Corn steep liquor | 0.2 |
| Yeast extract | 0.2 |
| Ammonium sulfate | 1.3 |
| Monobasic potassium phosphate | 0.05 |
| Dibasic potassium phosphate | 0.1 |
| Magnesium sulfate | 0.02 |
| Calcium carbonate | 2.0 |

Example 7

In a 200 ml-Erlenmeyer flasks containing 20 ml respectively of a sterilized first seed culture medium having the composition as shown in Table 4 were inoculated a platinum loopful of each of Escherichia coli IFO 3547 and 3E5 (IFO 15216, FERM BP3564) grown on a slant culture medium, followed by 24 hours' rotary shaking culture at 30°C at 220 rpm. To a 1000 ml-Erlenmeyer flasks containing 200 ml respectively of a sterilized second seed culture medium having the same composition were transplanted 20 ml of each of the first seed culture broth, followed by 24 hours'rotary shaking culture at 30°C at 220 rpm. The respective second seed culture broth (125 ml) was transplanted to a 5 $\ell$-jar fermentator containing 2.0 $\ell$ of a sterilized culture medium comprising 175 g of glucose, 5 g of corn steep liquor, 30 g of ammonium sulfate, 1.25 g of monobasic potassium phosphate, 2.5 g of dibasic potassium phosphate, 0.5 g of magnesium sulfate and 37.5 g of calcium carbonate (pH 7.0), followed by culture at 38°C with aeration (0.8 Vol/Vol/Min) while stirring (900 rpm).

Reduction in concentration of dissolved oxygen with growth of the microorganisms was compensated by stirring and aeration. Over a 25-hour period starting 5 hours after culture initiation, a sterilized aqueous solution containing 100 g of glucose, 400 g of DL-pantoic acid and 120 g of β-alanine was continuously added.

After 60 hours' culture, the final volume of each of the culture broths was 2.5 $\ell$. The amount of the accumulated D-pantothenic acid was 60 g/$\ell$ in the culture broth of Escherichia coli IFO 3547, whereas the amount of the accumulated D-pantothenic acid was 117.5 g/$\ell$ in the culture broth of Escherichia coli 3E5 (IFO 15216, FERM BP 3564).

The molar yield based on D-pantoic acid in the former was 51%, whereas that in the latter was 99.9%.

The above culture broth of Escherichia coli 3E5 was filtered. A portion of the obtained filtrate (200 ml) was subjected to a column filled with 200 ml of Diaion ® PK-216 (H type). The thus-obtained eluate was combined with the washings. The mixture was further passed through a column filled with 50 ml of Diaion ® PA-412 (OH type).

The thus-obtained eluate was combined with the washings. Calcium hydroxide was added to the mixture until pH became 5.1. The mixture was concentrated under reduced pressure up to 50 g in weight.

To the concentrate was added 200 ml of isopropanol. The mixture was heated to 40 to 60°C, followed by addition of 0.1 g of seed crystals of calcium D-pantothenate. The mixture was stirred softly for 1 hour at the same temperature, and then stirred softly at 5°C for 16 hours.

The resulting crystals were collected by filtration, washed with 50 ml of isopropanol and dried to yield 21.2 g of calcium D-pantothenate. The purity of the obtained crystals was 99%, and the optical purity thereof was 100%. $[\alpha]_D^{25}$= +27.7 (C=5, $H_2O$)

Table 4

| Composition of Liquid Culture Medium (pH 7.0) | |
| --- | --- |
| Composite | Concentration (%) |
| Glucose | 5.0 |
| Corn steep liquor | 0.2 |
| Ammonium sulfate | 1.3 |
| Monobasic potassium phosphate | 0.05 |
| Dibasic potassium phosphate | 0.1 |
| Magnesium sulfate | 0.02 |
| Calcium carbonate | 2.0 |

## Claims

1. A method for production of D-pantothenic acid or a salt thereof, which comprises bringing DL-pantoic acid or D-pantolactone, or a mixture thereof, and β-alanine into contact with a microorganism which is capable of producing D-pantothenic acid from DL-pantoic acid and β-alanine but substantially does not produce L-pantothenic acid, and, if desired, converting the free acid to a salt form.

2. A method as claimed in claim 1, wherein said microorganism is a microorganism belonging to the species Escherichia coli.

3. The method as claimed in claim 1, wherein said microorganism is Escherichia coli IFO 14237.

4. The method as claimed in claim 1, wherein said microorganism is a transformant of a plasmid harbouring a D-pantothenic acid-synthesizing enzyme-coding gene DNA.

5. The method as claimed in claim 4, wherein said microorganism is Escherichia coli KE3 or Escherichia coli 3E5.

6. The method as claimed in claim 4, wherein said gene DNA is obtained from Escherichia coli K-12 or Escherichia coli IFO 3547.

7. The method as claimed in claim 4, wherein said plasmid is pFV3 or pFV5.

8. A plasmid harbouring a D-pantothenic acid-synthesizing enzyme-coding gene DNA.

9. The plasmid as claimed in claim 8 which is pFV3 or pFV5.

10. A microorganism which is a transformant of a plasmid harbouring a pantothenic acid-synthesizing enzyme-coding gene DNA and which is capable of producing D-pantothenic acid.

11. The microorganism of claim 10 wherein said microorganism is capable of producing D-pantothenic acid from DL-pantoic acid and β-alanine, but substantially does not produce L-pantothenic acid.

12. The microorganism as claimed in claim 10 which is Escherichia coli KE3 (FERM BP-3563) or Escherichia coli 3E5 (FERM BP-3564).

Fig. 1

E : *Eco* RI
EV: *Eco* RV
P : *Pvu* II
B : *Bgl* II